# EUROPÄISCHE PATENTANMELDUNG

(11) **EP 4 552 635 A1**
(43) Veröffentlichungstag der Anmeldung: **14.05.2025**
(21) Anmeldenummer: 24211488.2
(22) Anmeldetag: 07.11.2024
(51) Int. Cl.: A61K 31/045, A61K 31/185, A61K 31/522, A61K 33/14, A61P 25/32

(54) **MITTEL ZUR VERBESSERUNG DES WOHLBEFINDENS NACH STARKEM ALKOHOLGENUSS**

(30) Priorität: 09.11.2023 DE 102023004550
(71) Anmelder: Vill, Katharina, 80809 München (DE)
(72) Erfinder: Vill, Katharina, 80809 München (DE)
(74) Vertreter: Maiwald GmbH

(57) **Zusammenfassung**

Der Einsatz von Elektrolyten, insbesondere Natrium- und Kaliumionen, kann in Kombination mit Koffein, Taurin und einer geringen Menge "Gegenalkohol" die negativen Auswirkungen des Alkoholkonsums mildern und das allgemeine Wohlbefinden der Betroffenen am nächsten Tag entscheidend und nachhaltig verbessern. Demnach schlägt die Erfindung ein Präparat vor, das als Elektrolyte die Kationen Natrium und Kalium, vorzugsweise mit Chlorid und Citrat als Gegenionen, Koffein, Taurin und eine geringe Menge Alkohol enthält. Das Präparat kann als Tablette oder Pulver in Kombination mit einer geringen Menge Alkohol, z.B. in Form von verdünntem Ethanol, etwas Bier oder einem handelsüblichen Schnaps oder Likör eingenommen werden; bevorzugt wird jedoch die Darreichung als wässrige Lösung, da die zusätzliche Einnahme von reichlich Wasser die Wirkung verbessert.

## Beschreibung

Der Kater, auch "Hangover" genannt, ist ein unangenehmer Zustand, der nach übermäßigem Alkoholkonsum auftritt. Er ist durch eine Reihe unangenehmer körperlicher und geistiger Symptome gekennzeichnet, die häufig am Tag nach dem Alkoholkonsum, der in der Regel am Abend stattgefunden hat, auftreten. Viele Menschen wünschen sich jedoch, bereits am Tag nach einem solchen exzessiven Alkoholkonsum wieder leistungsfähig zu sein und den geplanten Tagesaktivitäten uneingeschränkt nachgehen zu können. Empfindliche Menschen können auch schon nach geringem Alkoholkonsum am nächsten Tag Einschränkungen verspüren, die sie möglichst vermeiden möchten.

Ein Kater wird durch verschiedene Faktoren verursacht, u. a. durch die toxischen Nebenprodukte des Alkoholabbaus, durch Dehydration aufgrund der harntreibenden Wirkung des Alkohols, durch Schlafstörungen und durch die Beeinträchtigung des Stoffwechsels. Die Symptome sind von Person zu Person verschieden, können aber folgende bekannte Merkmale aufweisen
1. Kopfschmerzen: Kopfschmerzen sind eines der häufigsten Symptome eines Katers. Sie werden häufig durch die Erweiterung der Blutgefäße im Gehirn, Dehydrierung und eine gestörte Schlafqualität verursacht.
2. Müdigkeit und Erschöpfung: Alkohol kann den normalen Schlafzyklus stören, was zu Müdigkeit und Erschöpfung am nächsten Tag führt. Der Schlaf ist weniger erholsam, was zu einem allgemeinen Gefühl der Müdigkeit führen kann.
3. Übelkeit und Erbrechen: Alkohol kann die Magenschleimhaut reizen und den Magen-DarmTrakt beeinträchtigen, was zu Übelkeit und Erbrechen führen kann.
4. Gedächtnis- und Konzentrationsstörungen: Alkohol kann die Fähigkeit des Gehirns zur Informationsverarbeitung beeinträchtigen, was zu Gedächtnis- und Konzentrationsproblemen führen kann.
5. Licht- und Lärmempfindlichkeit: Ein Kater kann die Licht- und Lärmempfindlichkeit erhöhen, was zu einem allgemeinen Unwohlsein führen kann.
6. Stimmungsschwankungen: Alkohol kann die Neurotransmitter im Gehirn beeinflussen und zu Stimmungsschwankungen wie Reizbarkeit, Angst oder Depressionen führen.

Seit langem gibt es Vorschläge, wie die auftretenden Beschwerden gemildert oder beseitigt werden können. Dabei wird vor allem auf Hausmittel zurückgegriffen. Das bekannteste ist wohl das bayerische "Konterbier", aber auch Kopfschmerzmittel wie Acetylsalicylsäure in Kombination mit Kaffee/Koffein sowie eine Reihe von Multivitamin- und/oder Naturstoffpräparaten werden vorgeschlagen. Gegen Übelkeit und Kopfschmerzen ist Konterbier recht wirksam, hat aber den Nachteil, dass die Beschwerden nach Absetzen des Alkohols in der Regel wieder auftreten. Andere Hausmittel sind in der Regel unzureichend.

Phytopharmaka enthalten häufig Mariendistel (Silymarin), auch in Kombination mit Koffein und Taurin als Stimulanzien. Mariendistel soll toxische Leberschäden bei Medikamenteneinnahme und Alkoholkonsum reduzieren, siehe z.B. DE 10 2011 013224 A1, ist aber nicht kurzfristig wirksam. In CA 3012021 A1 wird Taurin eingesetzt, um die Enzyme des Alkoholstoffwechsels und der Entgiftung zu beschleunigen. Nach US 2002 0192303 A1 ist Taurin ein Osmo-Regulator; nach WO 01/91759 soll es zusammen mit einem Ca-Antagonisten, z.B. einem Mg-Salzkomplex oder einem Mg-Chelat, und einem Fettsäurebinder, z.B. Carnitin, eingesetzt werden.

Häufig wird auch vorgeschlagen, L-Cystein zur Bekämpfung des Hangovers einzusetzen, z.B. in Kombination mit Ascorbinsäure und Vitamin B3 (EP 346331 A1). Auch andere Proteine wie Kreatin sollen hilfreich sein, insbesondere auch in Kombination mit Taurin und Koffein sowie weiteren Substanzen und B-Vitaminen wie in WO 2019 081468 A1 beschrieben. EP1757289 A1 beschreibt die Verwendung von Koffein mit Peptiden wie Serin und Tryptophan.

In WO 00 2009 071385 A1 wird vorgeschlagen, dem Alkohol, in diesem Fall Wein, Taurin und Koffein bereits beim Trinken zuzusetzen, um trotz des Alkoholkonsums eine anregende Wirkung zu erzielen. Dies schützt jedoch keineswegs vor einem Kater, wenn dieses Getränk in größeren Mengen konsumiert wird.

Was in der Literatur weniger beachtet wird, ist die Tatsache, dass Alkohol ein harntreibendes Mittel ist, das dazu führt, dass der Körper mehr Flüssigkeit ausscheidet. Dies kann zu einer Dehydration führen, die Symptome wie Mundtrockenheit, Durstgefühl und Kopfschmerzen verstärken kann. Außerdem wird der Elektrolythaushalt gestört, wobei insbesondere ein Ungleichgewicht von Natrium, Kalium und Magnesium eine entscheidende Rolle spielt. Dies kann unter anderem zu einer Störung des Gleichgewichtssystems im Innenohr führen, was Schwindel und Schüttelfrost auslösen kann.

Eine absolut sichere Methode zur Vermeidung eines Katers gibt es nicht, da jeder Organismus anders reagiert. Am besten ist es jedoch, den Alkoholkonsum in Maßen zu halten, ausreichend Wasser zu trinken, beim Trinken auch alkoholfreie Getränke zu sich zu nehmen, ausreichend zu schlafen und auf eine ausgewogene Ernährung zu achten.

Die vorliegende Erfindung soll denjenigen helfen, die diese Ratschläge ignoriert haben, sich aber einen Kater oder Hangover am Tag nach starkem oder exzessivem Alkoholkonsum nicht leisten können oder wollen, oder denjenigen, die sehr empfindlich auf Alkohol reagieren oder dies befürchten und mögliche Nebenwirkungen vermeiden wollen. Überraschenderweise konnte gezeigt werden, dass der Einsatz von Elektrolyten, insbesondere Natrium- und Kaliumionen, in Kombination mit Koffein, Taurin und einer geringen Menge "Gegenalkohol" die negativen Auswirkungen des Alkoholkonsums mildern und das allgemeine Wohlbefinden der Betroffenen am nächsten Tag entscheidend und nachhaltig verbessern kann.

Die Erfindung schlägt ein Präparat vor, das als Elektrolyte die Kationen Natrium und Kalium, vorzugsweise mit Chlorid und Citrat als Gegenionen, Koffein, Taurin und eine geringe Menge Alkohol enthält. Das Präparat kann als Tablette oder Pulver in Kombination mit einer geringen Menge Alkohol, z.B. in Form von verdünntem Ethanol, etwas Bier oder einem handelsüblichen Schnaps oder Likör eingenommen werden; bevorzugt wird jedoch die Darreichung als wässrige Lösung, da die zusätzliche Einnahme von reichlich Wasser die Wirkung verbessert.

Mögliche Zusatzstoffe sind die im Lebensmittelbereich für Getränke üblichen Stoffe, wie z.B. Glucose, Saccharose, Propylenglykol, Aspartam, Benzylalkohol, L-Carnitin, Säuerungsmittel wie Zitronensäure, Citrate der Ionen Natrium und/oder Magnesium, die nicht nur wegen ihrer Elektrolyt-Funktion, sondern auch als Säuerungsregulatoren fungieren können, Vitamine (z.B. Vitamin C, Vitamin B6, Niacin, Pantothensäure), Aromastoffe, Farbstoffe, Riboflavin, Zuckercouleur, Konservierungsstoffe wie Benzoesäure sowie Kohlensäure, einzeln oder in beliebigen Kombinationen. Weitere Zusatzstoffe sind nicht ausgeschlossen.

Die Mengen der erfindungsgemäß notwendigen Komponenten sollten so eingesetzt werden, dass die wünschenswerte Wirkung erzielt wird. Die Gabe ist daher unter anderem unter Berücksichtigung des Körpergewichts zu wählen. Da unterschiedliche Personen außerdem unterschiedlich auf die genannten Komponenten reagieren, ist die Angabe einer konkreten Unter- oder Obergrenze nicht sinnvoll; entscheidend ist, dass die Komponenten in pharmakologisch wirksamer Weise zugesetzt werden. Mit der Nahrung werden täglich bei gesunder, gemischter Kost üblicherweise ca. 400 mg Taurin aufgenommen; die zusätzliche Einnahme mithilfe des erfindungsgemäßen Mittels kann bei sehr wenig (z.B. 50 mg), meist aber mindestens 200 mg bis zu ca. 6 g liegen; für eine Person mit ca. 70 kg Körpergewicht sind zumindest 3 g unbedenklich. Koffein ist ab ca. 1 mg/kg Körpergewicht ein psychischer Wachmacher; ab ca. 2,5 mg/kg Körpergewicht stellt sich eine Kreislaufanregung ein, die bis zu einer erregenden Wirkung gesteigert sein kann. Daher empfiehlt sich in der Regel die Gabe von ca. 15 bis ca. 500 mg Koffein. An Elektrolyten kann das erfindungsgemäße Mittel insbesondere 3 bis 40 mmol Natrium und 1 bis 12 mmol Kalium enthalten. Als Gegenionen für die Alkali-Ionen eignen sich u.a. Chlorid und/oder Citrat in beliebiger Kombination. In einer bevorzugten Ausführungsform werden hierfür 0,15 bis 1,5 g NaCl, 0,1 bis 1,0 g KCI und 0,0 bis 1,5 g Dinatriumhydrogencitrat x 1,5 H2O eingesetzt. Der Alkoholgehalt (Ethanolgehalt) kann für sehr empfindliche Personen bei 0,5 g, ansonsten bei 1 bis 25 g liegen.

Stärker bevorzugt ist es, dass das erfindungsgemäße Mittel Dosen von 0,6 bis 1,8 g Taurin und/oder 50 bis 200 mg Koffein enthält.

Unabhängig davon ist es ebenso stärker bevorzugt, dass das erfindungsgemäße Mittel etwa 8 bis 24 mmol Natrium und/oder etwa 2,5 bis 8 mmol Kalium, z.B. etwa 0,25 bis 0,80 g NaCl und/oder 0,20 bis 0,6 g KCI und/oder 0,2 bis 0,8 g Dinatriumhydrogencitrat x 1,5 H2O enthält.

Der Alkoholgehalt in allen vorgenannten Ausführungsformen liegt stärker bevorzugt bei 2,5 bis 20 g, noch stärker bevorzugt bei 10 bis 20 g und in einer gegenüber dieser noch stärker bevorzugten Ausführungsform bei 13 bis 17 g.

Die genannten Substanzen werden in all diesen Ausführungsformen vorzugsweise in einem drittel Liter oder einem halben Liter Flüssigkeit auf Wasserbasis gelöst verabreicht. Die Aufnahme der Lösung durch die Substanz erfolgt vorzugsweise am dem dem Abend mit Alkoholgenuss folgenden Vormittag, nach dem Aufstehen und vor oder nach einem Frühstück, soweit dieses schon vertragen wird. Als Wasserbasis kann Wasser selbst dienen, das eines oder mehrere der vorgenannten Zusatzstoffe enthalten kann. Anstelle von Wasser können auch beispielsweise Apfelsaft und/oder Gemüsesaft verwendet werden, wobei bei der Verwendung von Apfelsaft kaum oder kein Kalium zugesetzt werden muss, da ein solcher Saft ca. 120 mg Kalium pro 100 ml enthält, während bei der Verwendung von Gemüsesaft auf den Zusatz von weiterem Natrium verzichtet werden oder dieser gesenkt werden kann, da Gemüsesaft ca. 150 mg Natrium pro 100 ml enthält.

In einer ganz besonders bevorzugten Ausführungsform, die auch den nachfolgenden Beispielen 1 und 2 zugrunde liegt, wurden 0,47 g NaCl, 0,3 g KCI, 0,53 g Dinatriumhydrogencitrat x 1,5 H2O zusammen mit 3,6 g Glucose in 250 ml Wasser gelöst. Die Lösung wurde mit 150 ml eines Energy-Drinks gemischt, der ca. 0,4% Taurin (0,6 g absolut) und 0,03% (45 mg absolut) Koffein enthielt. Zu dieser wässrigen Lösung gab man eine alkoholische Lösung in einer solchen Menge, dass die Lösung etwa 4,5 Vol-% Alkohol enthielt, in diesem Falle 50 ml eines 38 Vol.-%igen Wodka.

Es ist möglich, eine zweite Darreichung in einer der vorgenannten Mengen bzw. Zusammensetzungen ca. 2 Stunden nach der ersten Verabreichung zu geben, vor allem, wenn die Mengen der genannten Substanzen eher im unteren Teil der angegebenen Bereiche angesiedelt sind.

Anhand der nachstehenden Beispiele und Vergleichsbeispiele kann gezeigt werden, dass das erfindungsgemäße Mittel mit Elektrolyten, Koffein, Taurin und einer geringen Menge "Gegenalkohol" dazu beitragen kann, einige der negativen Auswirkungen des Alkoholkonsums zu mildern und das allgemeine Wohlbefinden am nächsten Tag entscheidend und nachhaltig zu verbessern.

Der Kater wird in der Gesellschaft gerne mit dem oben erwähnten "Konterbier" bekämpft, da es durch die Erhöhung des Alkoholspiegels die Symptome lindert, oder mit dem Mischgetränk "Wodka-Energy-Drink", bei dem der Alkohol zusätzlich mit Koffein und Taurin (das vorübergehend die Müdigkeit bekämpft und die Wachsamkeit erhöht), kombiniert wird. Allerdings können sowohl das Konterbier als auch die Mischung von Alkohol und Koffein/Taurin den Körper zusätzlich belasten und das Dehydrationsrisiko erhöhen, da der Alkohol erneut diuretisch wirkt und in der Folge weiter Elektrolyte ausgeschieden werden, anstatt substituiert zu werden. Durch die Kombination von Alkohol mit oder ohne Wachmacher wird somit eine der Hauptursachen des "Katers" nicht angegangen, denn die durch den Alkoholkonsum verursachten Elektrolytstörungen bleiben unbehandelt.

Im Gegensatz dazu kann das Mittel der vorliegenden Erfindung nicht nur die Symptome vorübergehend unterdrücken, sondern den Körper aktiv bei der Wiederherstellung seiner Homöostase unterstützen. Die enthaltenen Salze sollen den gestörten Elektrolythaushalt wieder ins Gleichgewicht bringen, der für die Zellfunktion von entscheidender Bedeutung ist. Die Elektrolytkomponenten, insbesondere wenn sie in Form einer medizinischen Elektrolytlösung gegeben werden, helfen, die Folgen der Dehydrierung zu reduzieren, während Koffein und Taurin die Wachsamkeit fördern und die geringe Menge Alkohol das allgemeine Wohlbefinden steigert.

In den nachfolgend dargestellten Versuchsreihen konnte gezeigt werden, dass zwar auch die beiden herkömmlichen Methoden die Katersymptome am nächsten Morgen bis zu einem gewissen Grad reduzieren konnten, jedoch nur für kurze Zeit. Wurde die erneute Alkoholzufuhr wieder unterbrochen, stellte sich trotz Wasserzufuhr innerhalb weniger Stunden wieder ein Elendgefühl ein, das bei der Einnahme des erfindungsgemäßen Mittels nicht wieder auftrat.

Zusammenfassend bietet das vorgestellte Getränk eine Kombination aus hochwirksamer kurzfristiger Symptomkontrolle und mittelfristiger Dehydratationskontrolle.

### Beispiele:

Beispiel 1 findet im Rahmen eines Festivalbesuchs mit billigem Alkohol statt, Beispiel 2 im Rahmen einer Firmeneinladung mit teurem Alkohol. Beide Szenarien finden im "Feiermodus" statt, in dem die allgemein bekannten Regeln der Vernunft in Vergessenheit geraten. Beide Szenarien wurden zwar unter Überwachung durchgeführt, sie sind jedoch lebensnah.

### Beispiel 1:

In diesem Beispiel wurde der Einfluss von Wasser und dem erfindungsgemäßen Mittel in Form von 400 ml der oben genannten wässrigen Zusammensetzung, der 50 ml eines 38 Vol.-%igen Wodkas zugesetzt waren, auf das Wohlbefinden von drei verschiedenen Versuchspersonen untersucht, die nach exzessivem Alkoholkonsum einen starken "Kater" hatten. Die Versuchspersonen hatten am Abend zuvor Alkohol in den unten angegebenen Mengen konsumiert und tranken tags darauf zwischen 9 und 10 Uhr 0,5 Liter Wasser. Um 10 Uhr erhielten sie das genannte erfindungsgemäße Mittel. Die Veränderungen der Kopfschmerzintensität und des allgemeinen Wohlbefindens wurden um 9 Uhr, 10 Uhr und 11 Uhr jeweils mittels einer Visuellen Analogskala (VAS) beurteilt, wie sie auch von Krankenhäusern bei der Aufnahme von Patienten zur Einschätzung von Schmerzen genutzt wird. Zwischen 11 und 14 Uhr und zwischen 14 und 18 Uhr wurden nochmals jeweils 0,5 l Mineralwasser konsumiert, und das Ausmaß von Kopfschmerzen und Allgemeinbefinden wurde erneut um 14 und 18 Uhr mittels VAS erhoben.

Die VAS umfasst eine Skala von 0 bis 10, wobei die Kopfschmerzen mit abnehmenden Werten abnehmen, bezüglich der Befindlichkeit die Werte 0 und 1 Übelkeit und "Sterbenselendigkeit" wiederspiegeln und die steigenden Werte zunehmendes Wohlbefinden anzeigen.

### Versuchspersonen und generelles Trinkverhalten:

Person 1: Regelmäßiger Alkoholkonsum, männlich, 46 Jahre, 184 cm, 84 kg.
Person 2: Gelegentlicher Alkoholkonsum, männlich, 39 Jahre, 175 cm, 75 kg.
Person 3: Alkoholkonsum nur am Wochenende, dann teils exzessiv, weiblich, 32 Jahre, 176 cm, 65 kg.
Abendlicher Alkoholkonsum: 5 Augustiner Bier, 2 Gin Tonic, 5 Schnaps "Berliner Luft" von 16:00 Uhr bis 24:00 Uhr.
Nahrung: Um 20 Uhr 1 Döner Kebap

### Ergebnisse:

Alkoholkonzentrationen in der Atemluft, gemessen mittels "Alkotest": Die folgenden Ergebnisse (Tabelle 1) zeigen die Alkoholkonzentrationen in der Atemluft der Versuchspersonen zu den angegebenen Zeitpunkten, gemessen mittels Schnelltest in der Atemluft:

**Tabelle 1**

| **Zeitpunkt** | **Alkoholkonzentration (Promille)** - **Person 1** | **Alkoholkonzentration (Promille)** - **Person 2** | **Alkoholkonzentration (Promille)** - **Person 3** |
|---|---|---|---|
| 18:00 Uhr | 0,5 | 0,6 | 0,7 |
| 20:00 Uhr | 1,0 | 1,2 | 1,1 |
| 22:00 Uhr | 1,3 | 1,6 | 1,6 |
| 24:00 Uhr | 1,6 | 2,0 | 1,9 |
| 09:00 Uhr | 0,4 | 1,1 | 0,7 |

Die folgenden Tabellen zeigen die VAS-Werte im Kopfschmerzgrad (Tabelle 2) und Allgemeinbefinden (Tabelle 3) der Versuchspersonen um 9 Uhr, 10 Uhr, 11 Uhr, 14 Uhr und 18 Uhr des nachfolgenden Tages und damit deren Verlauf über den Tag:

**Tabelle 2**

| | 9 Uhr | 10 Uhr | 11 Uhr | 14 Uhr | 18 Uhr |
|---|---|---|---|---|---|
| Person 1 | 6 | 5 | 0 | 0 | 0 |
| Person 2 | 9 | 8 | 1 | 0 | 0 |
| Person 3 | 7 | 7 | 1 | 0 | 0 |

**Tabelle 3**

| | | | | | |
|---|---|---|---|---|---|
| Person 1 | 4 | 6 | 9 | 9 | 9 |
| Person 2 | 4 | 4 | 8 | 9 | 9 |
| Person 3 | 3 | 4 | 8 | 9 | 8 |

### Beispiel 2:

Analog zu Beispiel 1, dieselben Probanden mit folgendem Konsum:
2 Glas 0,1 l Franciacorta Rosé, 1 Flasche Sauvignon Blanc aus dem Friaul, 2 Williams Birnenschnaps, 1 Glas Amarone. 3-Gänge Menü zwischen 19:30 Uhr und 21:00 Uhr.

Kopfschmerzen und Allgemeinbefinden wurden erneut mithilfe einer Visuellen Analogskala (VAS) um 9 Uhr, 10 Uhr und 11 Uhr des Folgetags bewertet. Wobei um 9 Uhr 0,5 l Mineralwasser und um 10 Uhr dasselbe Elektrolyt-Taurin-Koffein-Wodka-Getränk wie in Beispiel 1 angeboten wurde.

### Ergebnisse

### Alkoholkonzentrationen in der Atemluft, gemessen mittels "Alkotest"

Die folgenden Ergebnisse (Tabelle 4) zeigen die Alkoholkonzentrationen in der Atemluft der Versuchspersonen zu den angegebenen Zeitpunkten, gemessen mittels Schnelltest in der Atemluft:

**Tabelle 4**

| **Zeitpunkt** | **Alkoholkonzentration (Promille)** - **Person 1** | **Alkoholkonzentration (Promille)** - **Person 2** | **Alkoholkonzentration (Promille)** - **Person 3** |
|---|---|---|---|
| 20:00 Uhr | 0,4 | 0,5 | 0,6 |
| 22:00 Uhr | 0,9 | 1,0 | 1,1 |
| 00:30 Uhr | 1,4 | 1,7 | 1,6 |
| 09:00 Uhr | 0,3 | 0,8 | 0,4 |

Die folgenden Tabellen zeigen die Veränderungen im Kopfschmerzgrad (Tabelle 5) und Allgemeinbefinden (Tabelle 6) der Versuchspersonen um 9 Uhr, 10 Uhr, 11 Uhr, 14 Uhr und 18 Uhr des Folgetags:

**Tabelle 5**

| | 9 Uhr | 10 Uhr | 11 Uhr | 14 Uhr | 18 Uhr |
|---|---|---|---|---|---|
| Person 1 | 3 | 3 | 0 | 0 | 0 |
| Person 2 | 4 | 3 | 0 | 0 | 0 |
| Person 3 | 4 | 4 | 0 | 0 | 0 |

**Tabelle 6**

| | | | | | |
|---|---|---|---|---|---|
| Person 1 | 6 | 6 | 9 | 10 | 9 |
| Person 2 | 5 | 5 | 8 | 9 | 9 |
| Person 3 | 5 | 5 | 8 | 9 | 10 |

Es zeigt sich also, dass die Kater der Versuchspersonen sowohl nach exzessivem Konsum von günstigem als auch von edlerem Alkohol wirksam bekämpft werden können.

### Vergleichsbeispiel 1:

Dieses Beispiel wurde durchgeführt, um das Befinden der Versuchspersonen nach Einnahme des erfindungsgemäßen Mittels mit dem Befinden dieser Personen zu vergleichen, wenn sie dieselbe Art und Menge Alkohol wie in Beispiel 1 beschrieben zu sich genommen hatten, am nächsten Vormittag jedoch klassisches "Konterbier" anstelle des erfindungsgemäßen Mittels tranken.

Die Versuchspersonen waren dieselben wie in Beispiel 1 und tranken dieselbe Art und Menge Alkohol wie in Beispiel 1.

Kopfschmerzen (Tabelle 7) und Allgemeinbefinden (Tabelle 8) wurden am nächsten Tag erneut mithilfe einer Visuellen Analog Skala (VAS) um 9 Uhr, 10 Uhr und 11 Uhr, sowie um 14 Uhr und um 18 Uhr bewertet. Wobei um 9 Uhr 0,5 I Mineralwasser angeboten wurden und um 10 Uhr 0,5 I Helles Vollbier (Augustiner).

**Tabelle 8**

| | 9 Uhr | 10 Uhr | 11 Uhr | 14 Uhr | 18 Uhr |
|---|---|---|---|---|---|
| Person 1 | 6 | 6 | 1 | 2 | 2 |
| Person 2 | 9 | 6 | 2 | 4 | 4 |
| Person 3 | 8 | 7 | 1 | 2 | 3 |

**Tabelle 9**

| | | | | | |
|---|---|---|---|---|---|
| Person 1 | 4 | 6 | 8 | 6 | 5 |
| Person 2 | 3 | 4 | 7 | 5 | 4 |
| Person 3 | 4 | 4 | 7 | 5 | 4 |

Es zeigte sich, dass zwar die Kopfschmerzen weitgehend (wenn auch nicht so stark wie im Falle der Einnahme des erfindungsgemäßen Mittels) zurückgingen, dass sich jedoch das allgemeine Wohlbefinden, das sich nach dem Biertrinken etwas verbessert hatte, im Laufe des Nachmittags wieder verschlechterte.

### Vergleichsbeispiel 2:

Dieses Beispiel wurde durchgeführt, um das Befinden der Versuchspersonen nach Einnahme des erfindungsgemäßen Mittels mit dem Befinden derselben Personen zu vergleichen, wenn sie dieselbe Art und Menge Alkohol wie in Beispiel 1 beschrieben zu sich genommen hatten, am nächsten Vormittag jedoch einen mit Wodka versetzten Energy-Drink anstelle des erfindungsgemäßen Mittels tranken.

Die Versuchspersonen waren dieselben wie in Beispiel 1 und tranken dieselbe Art und Menge Alkohol wie in Beispiel 1.

Kopfschmerzen (Tabelle 10) und Allgemeinbefinden (Tabelle 11) wurden am nächsten Tag erneut mithilfe einer Visuellen Analog Skala (VAS) um 9 Uhr, 10 Uhr und 11 Uhr, sowie um 14 Uhr und um 18 Uhr bewertet. Wobei um 9 Uhr 0,5 l Mineralwasser angeboten wurden und um 10 Uhr 0,33 l Wodka-Redbull (incl. 5 cl Wodka). Zwischen 11 und 14 Uhr und zwischen 14 und 18 Uhr wurden jeweils erneut 0,5 l Mineralwasser konsumiert.

**Tabelle 10**

| | 9 Uhr | 10 Uhr | 11 Uhr | 14 Uhr | 18 Uhr |
|---|---|---|---|---|---|
| Person 1 | 5 | 4 | 1 | 4 | 3 |
| Person 2 | 8 | 7 | 2 | 6 | 4 |
| Person 3 | 8 | 7 | 1 | 3 | 3 |

**Tabelle 11**

| | | | | | |
|---|---|---|---|---|---|
| Person 1 | 3 | 4 | 8 | 4 | 3 |
| Person 2 | 1 | 1 | 7 | 5 | 4 |
| Person 3 | 3 | 4 | 7 | 5 | 4 |

Aus diesem Vergleichsbeispiel wird ersichtlich, dass ohne die Gabe von Kalium- und Natrium-Elektrolyt zwar initial eine Besserung der Kopfschmerzen und des Allgemeinbefindens zu verzeichnen war, diese Besserung über den Tag hinweg jedoch nicht anhielt.

Zusammenfassend zeigen die Beispiele, dass die Kopfschmerzintensität und das allgemeine Wohlbefinden, mit der Visuellen Analogskala (VAS) gemessen, bei der Gabe einer Kombination von 0,5 I Wasser und dem erfindungsgemäßen Mittel der Beispiele 1 und 2 am nächsten Vormittag bei allen drei Probanden im Vergleich mit einer ausschließlichen Aufnahme von erwartbaren Trinkmengen Wasser signifikant verbessert waren und dass die Gabe des erfindungsgemäßen Mittels bei diesen Personen einen signifikant besseren Einfluss auf das Wohlbefinden im längeren Tagesverlauf als ein Konterbier oder ein "Wodka-Redbull" zur Katerbekämpfung besaß. Die individuellen Unterschiede zwischen den Testpersonen spiegeln dabei deren unterschiedliche Trinkgewohnheiten und Körperreaktionen wider.

Weitere Beispiele der Erfindung werden im Anschluss dargestellt:
Beispiel 1: Mittel zur Verbesserung des Wohlbefindens nach Alkoholgenuss, das als Einzeldosis Koffein und Taurin in jeweils pharmakologisch wirksamer Menge, mindestens 2 mmol Natriumionen, mindestens 1,5 mmol Kaliumionen und mindestens 0,5 g Alkohol enthält.
Beispiel 2: Mittel nach Beispiel 1, umfassend eine feste Komponente mit Koffein und Taurin sowie den genannten Natriumionen und Kaliumionen in Form von Salzen sowie getrennt davon den Alkohol in einer wasserhaltigen Lösung.
Beispiel 3: Mittel nach Beispiel 1, worin das Mittel als wässrige Lösung vorliegt, die alle Komponenten enthält.
Beispiel 4: Mittel nach Beispiel 3, das ein Volumen von 200 bis 800 ml besitzt.
Beispiel 5: Mittel nach einem der voranstehenden Beispiele, enthaltend
   mindestens 50 mg Taurin und
   mindestens 20 mg Koffein.
Beispiel 6: Mittel nach Beispiel 5, enthaltend
   0,1 bis 4 g Taurin und/oder
   15 bis 500 mg Koffein und/oder
   3-40 mmol Natriumionen und/oder
   2-12 mmol Kaliumionen und/oder
   1,0 bis 25 g Alkohol.
Beispiel 7: Mittel nach Beispiel 6, enthaltend
   0,6 bis 1,8 g Taurin und/oder
   35 bis 200 mg Koffein und/oder
   8-24 mmol Natriumionen und/oder
   2,5-8 mmol Kaliumionen und/oder
   10 bis 20 g Alkohol.
Beispiel 8: Mittel nach einem der Beispiele 1 bis 6, enthaltend 0,25 bis 0,80 g NaCl und 0,20 bis 0,6 g KCI und 0,2 bis 0,8 g Dinatriumhydrogencitrat x 1,5 H2O.
Beispiel 9: Mittel nach einem der voranstehenden Beispiele, zusätzlich enthaltend Glucose und/oder Saccharose.
Beispiel 10: Mittel nach einem der voranstehenden Beispiele, umfassend eine Mischung aus
   - 0,47 g NaCl, 0,3 g KCI, 0,53 g Dinatriumhydrogencitrat x 1,5 H2O mit 3,6 g Glucose in 250 ml Wasser
   - ca. 1,3 g Taurin und ca. 100 mg Koffein in ca. 150 ml eines zucker- und farbstoffhaltigen Getränks, das weitere Zusatzstoffe enthalten kann, sowie
   - ca. 50 ml eines ca. 38 Vol.-%igen alkoholischen Getränks.

## Patentansprüche

1. Mittel zur Verbesserung des Wohlbefindens nach Alkoholgenuss, das als Einzeldosis Koffein und Taurin in jeweils pharmakologisch wirksamer Menge, nämlich
0,1 bis 4 g Taurin und
15 bis 500 mg Koffein,
sowie
3-40 mmol Natriumionen und
2-12 mmol Kaliumionen und
1,0 bis 25 g Alkohol
enthält.

2. Mittel nach Anspruch 1, umfassend eine feste Komponente mit Koffein und Taurin sowie den genannten Natriumionen und Kaliumionen in Form von Salzen sowie getrennt davon den Alkohol in einer wasserhaltigen Lösung.

3. Mittel nach Anspruch 1, worin das Mittel als wässrige Lösung vorliegt, die alle Komponenten enthält.

4. Mittel nach Anspruch 3, das ein Volumen von 200 bis 800 ml besitzt.

5. Mittel nach einem der voranstehenden Ansprüche, enthaltend
0,6 bis 1,8 g Taurin und/oder
35 bis 200 mg Koffein und/oder
8-24 mmol Natriumionen und/oder
2,5-8 mmol Kaliumionen und/oder
10 bis 20 g Alkohol.

6. Mittel nach einem der Ansprüche 1 bis 4, enthaltend 0,25 bis 0,80 g NaCl und 0,20 bis 0,6 g KCI und 0,2 bis 0,8 g Dinatriumhydrogencitrat x 1,5 H2O.

7. Mittel nach einem der voranstehenden Ansprüche, zusätzlich enthaltend Glucose und/oder Saccharose.

8. Mittel nach einem der voranstehenden Ansprüche, umfassend eine Mischung aus
- 0,47 g NaCl, 0,3 g KCI, 0,53 g Dinatriumhydrogencitrat x 1,5 H2O mit 3,6 g Glucose in 250 ml Wasser
- ca. 1,3 g Taurin und ca. 100 mg Koffein in ca. 150 ml eines zucker- und farbstoffhaltigen Getränks, das weitere Zusatzstoffe enthalten kann, sowie
- ca. 50 ml eines ca. 38 Vol.-%igen alkoholischen Getränks.
